(19) Europäisches Patentamt

European Patent Office

Office européen des brevets

(11) **EP 1 656 956 A1**

(12) **DEMANDE DE BREVET EUROPEEN**

(43) Date de publication:
**17.05.2006 Bulletin 2006/20**

(21) Numéro de dépôt: **05300815.7**

(22) Date de dépôt: **13.10.2005**

(51) Int Cl.:
*A61L 15/28* (2006.01)    *A61K 8/73* (2006.01)
*A61K 8/02* (2006.01)    *A45D 44/00* (2006.01)
*A61J 1/00* (2006.01)    *A61M 35/00* (2006.01)
*B65D 81/32* (2006.01)

(84) Etats contractants désignés:
**AT BE BG CH CY CZ DE DK EE ES FI FR GB GR
HU IE IS IT LI LT LU LV MC NL PL PT RO SE SI
SK TR**
Etats d'extension désignés:
**AL BA HR MK YU**

(30) Priorité: **14.10.2004 FR 0410878**

(71) Demandeur: **L'OREAL**
**75008 Paris (FR)**

(72) Inventeur: **SIMON, Pascal**
**94320, THIAIS (FR)**

(74) Mandataire: **Tanty, François et al**
**Nony & Associés,**
**3, rue de Penthièvre**
**75008 Paris (FR)**

(54) **Produit destiné à être imprégné d'un liquide**

(57)    La présente invention concerne un dispositif (1)
comportant :

- un produit (4), notamment sous forme d'un support
fibreux, destiné à être imprégné d'un liquide et à être
appliqué sur le corps humain, comportant un matériau superabsorbant formant un gel après absorption du liquide,

- un réceptacle (2) comportant un logement (3) dans
lequel est reçu le produit avant l'utilisation, le volume
du logement (3) étant suffisant pour recevoir simultanément le produit et une quantité suffisante de liquide pour gélifier le produit (4).

FIG.1

EP 1 656 956 A1

## Description

La présente invention concerne notamment des produits destinés à être imprégnés d'un liquide et à être appliqués sur le corps humain, comportant un matériau superabsorbant formant un gel après absorption du liquide.

L'invention a trait également à des dispositifs et kits comportant de tels produits.

Les demandes WO 02/03898 et WO 03/033041 divulguent des pansements comprenant un matériau hydroabsorbant associé à une couche de renforcement ou une couche adhésive.

On connaît également par WO 03/022317 des pansements composés de matériau hydroabsorbant imprégné par une solution métallique, par exemple un sel d'argent. Ces pansements sont composés de fibres, tissées ou non-tissées, dérivées de cellulose ou d'alginate, seules ou en mélanges.

La demande WO 98/09590 décrit des pansements composés d'un mélange de fibres de cellulose modifiée avec d'autres fibres, par exemple des fibres en dérivés d'alginate.

EP 0 737 463 et EP 1 350 739 décrivent un dispositif agencé de manière à contenir, dans deux chambres, un produit à réhydrater, de type compresse textile ou éponge, et un liquide d'imprégnation. Les deux chambres sont reliées entre elles par un conduit à ouvrir lors de l'étape d'imprégnation.

US 2002/0192269 divulgue un dispositif comportant deux chambres, l'une contenant un produit pour le soin de la peau et l'autre contenant un liquide pour l'imprégnation du produit textile par immersion.

Il existe un besoin pour bénéficier de produits destinés à absorber un liquide et à être appliqués sur le corps humain, présentant un aspect attractif pour une application en cosmétologie, notamment, et pratiques à utiliser.

Il existe également un besoin de disposer de moyens permettant de réaliser aisément l'imprégnation d'un produit avant son application, avec le cas échéant une quantité prédéfinie de liquide.

Il existe encore un besoin pour bénéficier d'un produit comportant un matériau superabsorbant formant un gel après absorption d'un liquide, qui soit facile à manipuler et à disposer sur une surface du corps humain à traiter.

Il existe encore un besoin pour permettre un stockage et une conservation améliorés d'un produit comportant un matériau superabsorbant.

La présente invention vise à satisfaire à tout ou partie de ces besoins.

Selon l'un de ses aspects, la présente invention a pour objet un dispositif comportant un produit, notamment sous forme d'un support fibreux, destiné à être imprégné d'un liquide et à être appliqué sur le corps humain, comportant un matériau superabsorbant formant un gel après absorption du liquide et un réceptacle comportant un logement dans lequel est reçu le produit, avant l'utilisation, le volume du logement étant suffisant pour recevoir simultanément le produit et une quantité suffisante de liquide pour gélifier le produit.

L'invention permet de conserver facilement, à l'abri et au sec, le produit comportant le matériau superabsorbant, dans des conditions stériles ou non, tout en permettant à l'utilisateur de l'imprégner avec la quantité souhaitée de liquide.

De plus, la formation d'un gel peut permettre, le cas échéant, au produit de devenir translucide ou transparent, et d'offrir un aspect attrayant.

Selon un autre de ses aspects, l'invention a pour objet un kit comportant un produit, notamment sous forme d'un support fibreux, destiné à être imprégné d'un liquide et à être appliqué sur le corps humain, comportant un matériau superabsorbant formant un gel après absorption du liquide, et un liquide destiné à gélifier le produit.

Selon un autre de ses aspects, l'invention a encore pour objet un applicateur comportant un tube rempli d'au moins un liquide, un élément d'application destiné à être imprégné par le ou les liquide(s) contenu(s) dans le tube, cet élément d'application comportant un matériau superabsorbant. Le matériau superabsorbant peut former un gel après imprégnation par le ou les liquide(s).

Selon encore un autre de ses aspects, l'invention a pour objet un procédé de traitement cosmétique comprenant les étapes suivantes :

- imprégner d'un liquide un produit comportant au moins 80 % en masse d'un matériau superabsorbant autre que le polyvinylalcool, formant un gel après absorption du liquide, et
- mettre en contact une surface à traiter avec le produit ainsi gélifié, notamment une surface du corps dépourvue de lésions.

Dans la description et les revendications, "gel" désigne un solide ou pseudo-solide mou, obtenu par la combinaison d'au moins deux composants, dont l'un liquide peut constituer la partie majoritaire, par exemple de 70 à 99 %, de préférence de 90 à 99 % en poids par rapport au poids de l'ensemble constitué par le produit et le liquide. Les gels formés peuvent avoir d'excellentes qualités d'usage. Ils peuvent être discrets, et dans certains modes de réalisation ils peuvent être transparents. Ils peuvent avoir d'excellentes propriétés mécaniques et être parfaitement adaptables aux différentes formes du visage et du corps. Ils peuvent être suffisamment résistants, cohésifs, flexibles et dotés d'une certaine élasticité pour permettre une manipulation aisée et éventuellement suffisamment adhésifs pour permettre une application en différents endroits du corps humain afin par exemple de libérer des actifs, notamment des actifs cosmétiques et/ou dermatologiques.

L'invention a encore pour objet, indépendamment ou en combinaison avec ce qui précède, un produit destiné à être imprégné d'un liquide et à être appliqué sur le corps humain, comportant au moins 80 % en poids d'un matériau superabsorbant par rapport au poids total du produit, autre que le polyvinylalcool, formant un gel après absorption du liquide.

L'invention, indépendamment ou en combinaison avec ce qui précède, a encore pour objet un dispositif comportant :

- un produit, notamment sous forme d'un support fibreux, destiné à être imprégné d'un liquide et à être appliqué sur le corps humain, comportant un matériau superabsorbant formant un gel après imprégnation avec le liquide, le produit devenant alors translucide ou transparent, et
- un réceptacle comportant un logement dans lequel est reçu le produit.

L'invention, indépendamment ou en combinaison avec ce qui précède, a encore pour objet un dispositif comportant :

- un produit en feuille destiné à être gélifié au moyen d'un liquide et à être appliqué sur le corps humain,
- un réceptacle comportant un logement dans lequel est reçu le produit, ce logement présentant un volume suffisant pour contenir la quantité de liquide nécessaire pour la gélification du produit en feuille, le réceptacle étant dépourvu de réservoir contenant ledit liquide avant l'étape de gélification.

L'invention, indépendamment ou en combinaison avec ce qui précède, a encore pour objet un kit comportant :

- un produit destiné à être imprégné d'un liquide et à être appliqué sur le corps humain, comportant au moins 80 % en poids d'un matériau superabsorbant formant un gel après absorption d'un liquide, et
- le liquide destiné à gélifier le produit.

L'invention a également pour objet, indépendamment ou en combinaison avec ce qui précède, un applicateur comportant :

- un tube rempli d'au moins un liquide,
- un élément d'application destiné à être imprégné par le liquide et comportant un matériau superabsorbant.

Le matériau superabsorbant convenant à la mise en oeuvre d'un tel applicateur est avantageusement un matériau superabsorbant sélectionné parmi ceux énumérés dans la description ci-après.

L'invention a également pour objet, indépendamment ou en combinaison avec ce qui précède, un procédé de traitement cosmétique comportant les étapes de :

- verser dans le logement d'un dispositif tel que défini précédemment un liquide pour imprégner un produit, et
- mettre en contact une surface à traiter avec ledit produit ainsi imprégné.

## Matériau superabsorbant

Le matériau superabsorbant présent dans le produit présente avantageusement une très forte capacité d'absorption d'un liquide, et notamment de l'eau. Notamment, il peut présenter la capacité d'absorber au moins 15 fois, voire 20 ou 50 fois son poids en eau, par exemple de l'ordre de 25 à 30 fois.

Selon un mode de réalisation, le matériau superabsorbant convenant à la mise en oeuvre de l'invention est anhydre.

Au sens de la présente invention, on entend désigner par « anhydre » un composé contenant moins de 5 %, en particulier moins de 3 %, en particulier moins de 2 %, et plus particulièrement moins de 1 % d'eau par rapport au poids total du matériau.

Il est possible de déterminer la capacité d'absorption du matériau superabsorbant selon la méthode suivante.

Un échantillon du matériau superabsorbant, à l'état de poudre, de fibres en vrac, ou agencées à l'état de film ou de feuille est pesé à l'état sec ($M_S$). Par exemple, il est possible d'utiliser un voile non-tissé, carré, d'environ 1 cm de côté. Dans le cadre de la présente méthode, le matériau superabsorbant est notamment obtenu à l'état "sec" après traitement dans une étuve à dessiccation pendant environ 4 heures, à environ 50 °C.

De l'eau (ou tout autre liquide destiné à être absorbé par le matériau) est mise en contact avec le matériau. Cette mise en contact peut s'effectuer soit par immersion du matériau dans le liquide, soit par versement de ce dernier sur le matériau. A titre d'exemple, le matériau peut être immergé pendant une durée d'une minute, environ.

La quantité d'eau (ou de liquide) est par exemple utilisée en excès de manière à saturer complètement le matériau. L'excès d'eau (ou de liquide) est par la suite éliminé, par exemple par égouttement pendant environ 2 minutes, et le matériau saturé en liquide est pesé ($M_L$).

La différence $\Delta$ entre le poids du matériau saturé en liquide et le poids du matériau sec, traduisant la quantité de liquide absorbée, est ensuite rapportée au poids sec du matériau. La valeur obtenue C est indicative de la capacité d'absorption du matériau superabsorbant, exprimée par exemple en gramme de liquide absorbé par gramme de matériau sec :

$$C = \frac{\Delta}{M_S} = \frac{(M_L - M_S)}{M_S}$$

Le produit peut, selon la proportion de matériau superabsorbant qu'il contient, absorber par exemple au moins 15 fois son propre poids du liquide, voire au moins 20 ou 50 fois son poids du liquide, de préférence de 25 à 30 fois.

La capacité d'absorption du produit comportant le matériau superabsorbant peut se déterminer de manière similaire au protocole évoqué précédemment pour le matériau superabsorbant.

Le matériau superabsorbant peut être choisi parmi les dérivés de cellulose, les alginates et leurs dérivés, notamment leurs dérivés tels que l'alginate de propylène glycol, ou leurs sels comme l'alginate de sodium, l'alginate de calcium, les dérivés d'acide polyacrylique ou polyméthacrylique, les dérivés de polyacrylamide, les dérivés de polyvinylpyrrolidonne, les dérivés d'éther polyvinylique, et leurs mélanges, entre autres.

Le matériau superabsorbant peut être notamment choisi parmi des dérivés de cellulose chimiquement modifiée. Il peut être, par exemple, choisi parmi la carboxyméthylcellulose, la carboxyméthylcellulose sodique, la carboxyméthyl-hydroxyéthylcellulose, la carboxyéthylcellulose, l'hydroxyéthylcellulose, l'hydroxyéthyl-éthylcellulose, l'hydroxypropylcellulose, l'hydroxypropylméthylcellulose, la méthylcellulose, la méthylcellulose sodique, la cellulose microcristalline, le sulfate de cellulose sodique et leurs mélanges.

Il peut être également choisi parmi les celluloses alkylées. Ces polymères sont obtenus par le greffage d'un reste alkyle sur un ou plusieurs groupements hydroxy du polymère cellulosique pour former le dérivé hydroxyalkylé. Ces restes alkyles peuvent être choisis parmi les groupements stéaryle, isostéaryle, lauryle, myristyle, cétyle, isocétyle, cocoyle, palmityle, oléyle, linonéyle, ricinoléyle, béhényle et leurs mélanges. Ces dérivés cellulosiques hydroxyalkylés peuvent encore subir une modification chimique par, par exemple, un reste carboxylique.

Il peut être également choisi parmi des dérivés polymériques naturels comme par exemple la gélatine et les polysaccharides glucomannanes et galactomannanes extraits des graines, des fibres de végétaux, des fruits, des algues marines, de l'amidon, des résines de plantes, ou encore d'origine microbienne. Il peut être choisi par exemple parmi la gomme d'agarose, la gomme de guar, la gomme adragante, la gomme de carrageenan, la gomme de konjac, la locust bean gum, la gomme gellan, la gomme de xanthane, et leurs mélanges.

Dans un exemple de mise en oeuvre de l'invention, le matériau superabsorbant est un dérivé de cellulose, chimiquement modifiée, notamment la carboxyméthylcellulose.

Des fibres en un matériau superabsorbant sont décrites dans les demandes WO 93/12275, WO 94/16746 et WO 00/01425, dont le contenu est incorporé par référence. Les fibres de carboxyméthylcellulose sont avantageusement obtenues en faisant réagir des fibres de cellulose en présence d'un agent alcalin fort, tel que l'hydroxyde de sodium, et un agent carboxyméthylant, tel que l'acide chloroacétique ou un sel de celui-ci tel qu'un sel de sodium. Avantageusement, le degré de substitution des fibres de cellulose obtenu est d'environ 0,1 à 0,5 groupe de carboxyméthyle par unité de glucose, voire de 0,2 à 0,4 groupe carboxyméthyle par unité de glucose. Des fibres en un matériau superabsorbant sont par exemple connues sous les dénominations commerciales Hydrofiber Technology et commercialisées par la société CONVATEC.

Dans un exemple de réalisation, le matériau superabsorbant comporte des fibres. Ces fibres peuvent être tissées ou liées entre elles autrement que par tissage, par exemple par un procédé de liage utilisé pour la fabrication des non-tissées.

Les fibres peuvent, par exemple, être au départ peignées par une cardeuse. Cette étape d'orientation des fibres peut conditionner les propriétés mécaniques de la feuille de fibres non-tissées. La feuille obtenue peut être par la suite consolidée par un traitement de type liage, pouvant s'effectuer de manière mécanique, thermique ou chimique. Des exemples de ce traitement sont l'aiguilletage ou l'enchevêtrement par jets d'un fluide, gaz ou liquide, compatible avec la nature des fibres.

Dans un autre mode de réalisation, la réaction de carboxyméthylation peut être conduite sur des non-tissés de fibres de cellulose, fabriqués préalablement à la réaction de carboxyméthylation par tout procédé connu d'obtention des non-tissés, et dans un mode préféré par cardage puis consolidation du voile par aiguilletage ou par jets d'eau. Ce mode de réalisation sera préféré si l'on souhaite partir d'un non-tissé obtenu par jets d'eau qui permet d'obtenir des structures plus denses que celles obtenues par aiguilletage. Ce mode de réalisation est notamment décrit dans la demande WO 94/16746, incorporée ici par référence.

A titre d'exemple de non-tissé convenant à la mise en oeuvre de l'invention, on peut utiliser ceux présentant une densité de fibres d'environ 50 g/m$^2$.

A titre d'exemple d'aiguilleté convenant à la mise en oeuvre de l'invention, on peut utiliser ceux présentant une densité de fibres d'environ 90 g/m$^2$.

Les fibres en matériau superabsorbant peuvent constituer au moins 80 % en poids du produit avant imprégnation par le liquide, voire plus. Par exemple, elles peuvent constituer au moins 90 %, voire au moins 99 % en poids du produit avant imprégnation par le liquide. Le produit peut encore être constitué essentiellement de telles fibres. Une proportion de fibres élevée peut faciliter l'obtention d'un effet attractif sur le plan visuel au moment de l'utilisation, le produit passant d'un état opaque à un état translucide ou transparent.

Selon une variante de réalisation, le produit peut comporter un dérivé de cellulose en une teneur d'au moins 0,1 %, notamment au moins 10 %, voire 50 % ou 80 %, voire sensiblement égale à 100 % du poids par rapport au poids total du produit.

Actifs

Le produit peut contenir au moins un actif. Eventuellement, le liquide destiné à l'imprégnation du produit peut également comporter un actif Ce ou ces actifs peuvent être sélectionnés, par exemple, parmi les actifs susceptibles d'agir sur la peau, tels que les actifs cosmétologiques et/ou dermatologiques.

Lorsque le produit comporte un actif, celui-ci peut être incorporé au produit notamment au moyen d'un champ électrique alternatif. Le ou les actifs peuvent être incorporés, notamment, à l'état pulvérulent.

Parmi l'ensemble des actifs utilisables dans la présente invention figurent, notamment, les acides $\alpha$- ou $\beta$-hydroxy, tels que l'acide lactique, l'acide glycolique, l'acide citrique, l'acide 5-octanoylsalicylique, l'acide $\alpha$-hydroxydécanoïque, l'acide $\alpha$-hydroxylaurique, l'acide tartarique, l'acide glucuronique, l'acide galacturonique, l'acide acrylique, l'acide $\alpha$-hydroxybutyrique, l'acide $\alpha$-hydroxyisobutyrique, l'acide malique, l'acide mandélique, l'acide phosphorique, l'acide pyruvique, l'acide lactobionique et l'acide salicylique.

Comme exemples d'autres actifs utilisables dans le cadre de la présente invention, il peut également être fait mention d'huiles cosmétiques, telles que les huiles de silicone, les huiles végétales de type triglycérides, les huiles hydrocarbonées telles que l'huile de parleam et les esters d'acides gras et d'alcool gras, permettant de conférer au gel des propriétés émollientes.

Il est également possible d'utiliser des actifs anti-acnéiques, tels que l'acide salicylique ou le péroxyde de benzoyle, l'octopirox, les acides aminés soufrés dextrogyres et lévogyres, leurs sels et leurs dérivés N-acétylé tels que la N-acétylcystéine, ou des actifs visant à prévenir le vieillissement de la peau et/ou à améliorer son état, par exemple les acides $\alpha$- et $\beta$-hydroxy cités précédemment, les rétinoïdes comme l'acide rétinoïque, le rétinol et ses esters comme par exemple le propionate de rétinyle, l'acétate de rétinyle ou le palmitate de rétinyle, la niacinamide, l'allantoïne, les extraits d'aloe, l'acide azelaique, le bisabolol, l'acide phytique, le collagène, ou des actifs stimulant la formation de collagène, des vitamines telles que la vitamine C ou des dérivés de celle-ci, comme l'ascorbyle glucoside, la vitamine E ou des dérivés de celle-ci, la vitamine A ou des dérivés de celle-ci, la vitamine F ou des dérivés de celle-ci, les acides aminés soufrés dextrogyres et lévogyres, et leurs dérivés tels que cités précédemment, l'élastine, la N-acétyl D-glucosamine, la lutéoline, ou des antioxydants tels que le thé vert ou des fractions actives de celui-ci, la glycérine, la laponite, la caféine, des huiles essentielles aromatiques, des colorants, des agents anti-radicaux libres, des actifs hydratants, des actifs dépigmentants, des agents améliorant le teint de la peau comme les agents de bronzage artificiel de type dihydroxyacétone ou ester de tyrosine, des liporégulateurs, des actifs adoucissants, antirides, kératolytiques, rafraîchissants, déodorants, insensibilisants, nourrissants et leurs mélanges. Il est également possible d'utiliser des actifs blanchissants, tels que l'acide kojique, des phosphates d'ascorbyle, des glucosides d'ascorbyle, de l'acide ascorbique et leurs mélanges.

Il peut également être possible d'utiliser des actifs permettant d'améliorer l'état de la peau, tels que des agents humidifiants ou des actifs permettant d'améliorer la barrière lipidique naturelle, tels que des céramides, des sulfates de cholestérol et/ou des acides gras et leurs mélanges. Il peut également être possible d'utiliser des enzymes ayant une activité sur la peau, telles que des protéases, des lipases, des cérébrosidases et/ou des mélanases et leurs mélanges.

Comme autres exemples d'actifs susceptibles de convenir à la mise en oeuvre de la présente invention figurent des actifs analgésiques, anesthésiques, anti-bactériens, anti-levures, anti-fongiques, anti-viraux, anti-dermatites, anti-pruritiques, anti-hémétiques, protecteurs vasculaires, contre le mal des transports, anti-irritants, anti-inflammatoires, immunomodulateurs, anti-hyperkératolytiques, pour le traitement de la peau sèche, anti-transpirants, anti-psoriatiques, anti-séborrhéiques, contre le vieillissement, antiasthmatiques et bronchodilateurs, protecteurs contre les UV, anti-histaminiques, cicatrisants, des cortico-stéroïdes, des actifs bronzants et leurs mélanges.

La teneur en actifs dans le produit et/ou dans le liquide pourra être ajustée en fonction de la région traitée et du but poursuivi. Elle peut, par exemple, être supérieure à 100 %, par exemple d'environ 150 %, en poids d'actif par rapport au poids du produit sans actif.

Liquides

Le liquide utilisé pour imprégner le produit est, par exemple, majoritairement constitué d'eau, en proportion variant entre 10 % et 100 % de la composition du liquide, de préférence entre 50 % et 100 %, encore de préférence entre 90 % et 100 %. Il peut être, par exemple, de l'eau, une solution aqueuse, une lotion hydroalcoolique ou alcoolique ou une émulsion. Lorsque l'utilisateur utilise de l'eau pour imprégner le produit, celle-ci peut être, par exemple, de l'eau du robinet, de l'eau de mer, de l'eau minérale ou de l'eau de source.

La viscosité du liquide mesurée au moyen d'un viscosimètre Brookfield Digital, modèle DV II, corps de mesure n° 3, pourra être comprise entre 100 mPa.s et 500000 mPa.s, de préférence entre 500 mPa.s et 100000 mPa.s, de préférence entre 500 mPa.s et 20000 mPa.s, de préférence entre 500 mPa.s et 3000 mPa.s.

Dans un exemple de mise en oeuvre de l'invention, le produit comporte, avant son imprégnation par un liquide, un ou plusieurs actifs choisis, par exemple, parmi ceux mentionnés précédemment. Le liquide utilisé pour gélifier le produit peut alors être dépourvu d'actifs et sera choisi de manière à solubiliser les actifs présents dans le produit.

Dans un autre exemple de mise en oeuvre de l'invention, à la fois le produit et le liquide destiné à l'imprégner comportent chacun un actif, par exemple choisi parmi les actifs mentionnés précédemment. Ce mode de réalisation est particulièrement avantageux lorsqu'il s'agit d'associer des actifs dont la combinaison peut être instable dans le temps. Ainsi, en séparant ces actifs dans le produit et dans le liquide destiné à gélifier le produit, leur conservation est améliorée.

Dans un autre exemple encore de mise en oeuvre de l'invention, le produit est dépourvu d'actifs et le liquide destiné à l'imprégnation du produit peut alors en comporter, le cas échéant.

Formes du produit

Le produit comportant un matériau superabsorbant peut être agencé sous la forme d'au moins une feuille, notamment lorsqu'il comporte ou est constitué essentiellement de fibres, par exemple de carboxyméthylcellulose ou de l'un des autres matériaux superabsorbants mentionnés ci-dessus.

Dans un exemple de mise en oeuvre de l'invention, le produit se présente sous la forme d'une feuille comportant des fibres non-tissées de matériau superabsorbant.

Selon un autre exemple de mise en oeuvre de l'invention, le produit peut être agencé sous forme d'une feuille comportant des fibres tissées.

Dans une variante de réalisation, le produit comporte une poudre et, le cas échéant, un filet. Ce dernier peut contribuer, par exemple, à la cohésion du produit après imprégnation par le liquide et transformation du matériau superabsorbant en gel.

Le produit peut présenter une structure monocouche comportant un matériau superabsorbant.

Le cas échéant, le produit peut présenter une structure multicouche, dont au moins une couche comporte le matériau superabsorbant. Les différentes couches peuvent, par exemple, comporter des actifs différents. Les différentes couches peuvent toutes comporter un matériau superabsorbant ou non. L'une des couches peut notamment être dépourvue de matériau superabsorbant, comportant par exemple des fibres, un fil, un film, un filet ou une charge inerte vis-à-vis du liquide ou n'en absorbant sensiblement pas. Le produit peut, par exemple, comporter une armature ou un support, utile pour renforcer la solidité et la résistance à la désagrégation du gel obtenu par imprégnation du produit.

Avantageusement, le produit selon l'invention est dépourvu de couche adhésive.

Lorsqu'il est présent, le support peut encore, par exemple, comporter une couche de fibres cellulosiques, ou d'une couche de fibres synthétiques telles que des fibres de polyamide, des fibres de polyester ou de polyéthylène.

Le produit peut, suite à l'absorption du liquide, présenter une diminution de sa surface extérieure. Ce rétrécissement peut être d'au moins 10 %, voire 40 % de la surface initiale.

Le produit peut présenter une augmentation de son épaisseur d'au moins 50 %, et notamment jusqu'à plus de 150 %, de son épaisseur initiale.

Le produit présente avantageusement, après imprégnation avec le liquide, une structure homogène et stable dans la période de temps nécessaire à son utilisation. Cette période de temps peut être comprise entre quelques secondes (par exemple, environ 10 secondes) et quelques heures (par exemple, environ 4 à 8 heures).

Le produit peut, avantageusement, après imprégnation avec le liquide, devenir transparent ou translucide.

Le produit, après imprégnation avec le liquide, peut être sensiblement non élastiquement déformable.

Le produit peut se présenter sous la forme d'une feuille découpée, par exemple sous la forme d'un masque ou d'un patch destiné à être appliqué sur le visage, ou toute autre partie du corps à traiter.

Le produit peut encore comporter une cavité dans laquelle peut être engagée au moins une partie du corps humain. Le produit peut présenter, par exemple, la forme d'un gant, notamment d'une moufle, permettant ainsi le traitement d'une main, ou son utilisation comme un gant pour traiter d'autres parties du corps.

Le produit peut, avantageusement, être présenté sous des formes variées, chacune adaptée à une utilisation particulière. Ainsi, il peut être présenté sous la forme d'un doigt de gant, d'un bonnet destiné à coiffer la tête afin de traiter les cheveux ou le cuir chevelu, d'une poche destinée à être disposée autour d'une oreille.

La cavité peut être réalisée à partir d'une feuille unique, repliée sur elle-même ou à partir de plusieurs feuilles assemblées entre elles, par soudure, collage ou couture par exemple.

Lorsque le produit est destiné à être appliqué sur au moins une partie du visage, il peut présenter au moins une ouverture, par exemple pour les yeux, le nez et/ou la bouche.

Réceptacle et organe de fermeture

Le dispositif comporte avantageusement un organe de fermeture pour fermer, notamment de manière étanche, le logement.

L'organe de fermeture peut comporter un opercule, lequel peut être fixé au réceptacle par collage ou thermosoudage, par exemple.

L'opercule peut comporter, par exemple, un film de matière plastique et/ou métal. L'opercule peut être, par exemple, un film de PET ou d'une polyoléfine, recouvert ou non d'un métal, par exemple d'aluminium.

Au moins une portion de l'opercule disposée en périphérie peut ne pas être adhérente au réceptacle afm de permettre une préhension de l'opercule, facilitant son enlèvement.

Le réceptacle peut comporter une plaquette.

Le logement peut présenter une paroi de fond plane et une paroi latérale inclinée, par exemple s'évasant en éloignement de la paroi de fond.

La paroi latérale est, par exemple, agencée de manière à suivre le contour du produit disposé dans le logement.

Le logement peut avantageusement présenter une profondeur supérieure à l'épaisseur maximale du produit. Cette profondeur est, par exemple, comprise entre 2 mm et 6 mm.

Le logement peut, par exemple, définir, notamment en fonction du produit à imprégner, un volume allant de 1 à 50 ml, notamment de 3 à 30 ml, voire de 4 à 20 ml.

Le logement contenant le produit peut être l'unique logement du réceptacle.

Lorsque le réceptacle comporte une pluralité de logements, chacun comportant un produit, ces produits peuvent être imprégnés avec ou comprendre des actifs respectifs différents. L'utilisateur peut alors, par exemple, choisir le produit en fonction du traitement à effectuer, ou utiliser plusieurs produits de forme différente, combinés à des liquides différents pour traiter simultanément des zones différentes du visage ou du corps.

Le réceptacle peut être réalisé au moins partiellement en matériau imperméable au liquide, par exemple une matière plastique et/ou un métal. Le métal peut être, par exemple, l'aluminium. A titre d'exemple de matière plastique, on peut citer les thermoplastiques, par exemple les polyoléfines notamment le polyéthylène ou le polypropylène. Le réceptacle peut être obtenu par thermoformage.

Le réceptacle est avantageusement constitué d'un matériau plastique au moins partiellement transparent ou translucide.

Le dispositif peut comporter une pluralité de produits. Dans une telle configuration, au moins deux produits peuvent comporter des actifs différents. Ces produits peuvent être compris dans des logements différents du réceptacle, comme indiqué ci-dessus.

Selon une variante de réalisation, les produits peuvent être disposés dans des logements différents appartenant à différents réceptacles, lesquels peuvent être reliés entre eux par des zones sécables, par exemple.

Kits

L'invention, indépendamment ou en combinaison avec ce qui précède, a encore pour objet un kit comportant :

- un produit, notamment tel que défini ci-dessus, destiné à être imprégné d'un liquide et à être appliqué sur le corps humain, comportant un matériau superabsorbant formant un gel après absorption du liquide, et
- le liquide destiné à imprégner ce produit.

L'invention a également pour objet, indépendamment ou en combinaison avec ce qui précède, un kit comportant :

- un produit destiné à être imprégné d'un liquide et à être appliqué sur le corps humain, comportant un matériau superabsorbant formant un gel après absorption du liquide,
- un dispositif comportant :
- un réceptacle comportant un logement destiné à recevoir ledit produit,
- un organe de fermeture du logement, et
- le liquide destiné à imprégner le produit.

Le kit peut comporter une pluralité de dispositifs. Ces dispositifs peuvent être contenus indépendamment les uns des autres dans un même conditionnement, ou associés au moins deux à deux au moyen de zones sécables, par exemple.

Lorsque le kit comporte une pluralité de produits, au moins deux produits peuvent comporter des actifs différents.

Le kit peut également comporter une pluralité de liquides. Ces liquides peuvent être différents.

Le ou chaque liquide destiné à être mis en oeuvre dans les kits peut être conditionné en récipient monodose.

Exemples

L'invention pourra être mieux comprise à la lecture de la description détaillée qui va suivre, d'exemples de mise en oeuvre non limitatifs de celle-ci, et à l'examen du dessin annexé, sur lequel :

- la figure 1 est une vue schématique en perspective d'un dispositif réalisé conformément à un exemple de mise en oeuvre de l'invention,
- la figure 2 est une section transversale, schématique et partielle, selon II-II de la figure 1,
- les figures 3 à 5 illustrent différents exemples de kits réalisés conformément à l'invention,
- la figure 6 est une vue analogue à la figure 2 d'une variante de mise en oeuvre de l'invention,
- la figure 7 est une vue de face d'une variante de réalisation d'un produit conforme à l'invention,
- la figure 8 est une section transversale selon VIII-VIII de la figure 7,
- la figure 9 est une vue de face d'une variante de réalisation d'un produit conforme à l'invention,
- la figure 10 est une vue de face d'une variante de réalisation d'un dispositif conforme à l'invention, comprenant le produit représenté à la figure 9,
- la figure 11 est une section transversale selon XI-XI de la figure 10, et
- la figure 12 représente de manière schématique, en élévation, un applicateur réalisé conformément à un autre exemple de mise en oeuvre de l'invention.

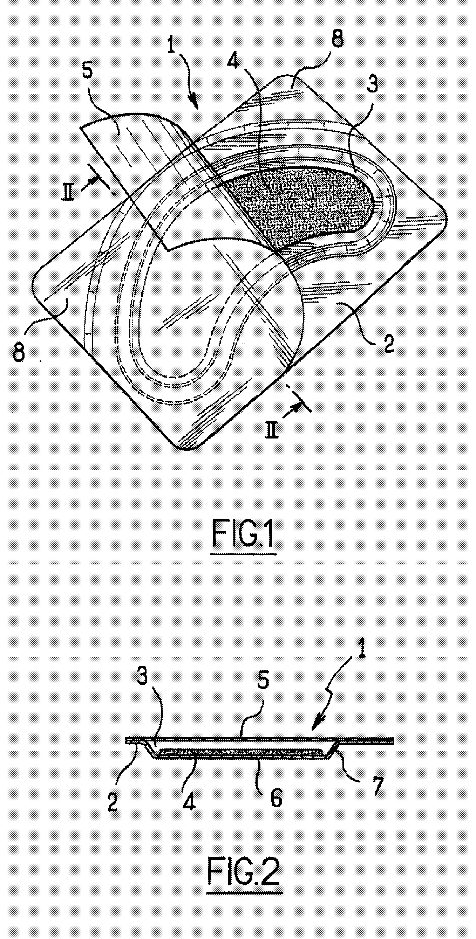

On a représenté aux figures 1 et 2 un exemple de dispositif 1, réalisé conformément à l'invention, comportant un réceptacle 2 comportant un logement 3 dans lequel est reçu un produit 4 à appliquer sur le corps humain, le réceptacle 2 étant fermé de manière étanche avant l'utilisation par un organe de fermeture 5, lequel se présente dans l'exemple considéré sous la forme d'un opercule.

Le logement 3 présente une paroi de fond 6 qui est sensiblement plane et une paroi latérale 7 qui épouse sensiblement le contour du produit 4 et qui va en s'évasant en éloignement de la paroi de fond 6.

L'opercule 5 est fixé au réceptacle 2 par exemple par thermosoudage ou thermocollage, sauf en au moins une portion 8 recouvrant une région du réceptacle en légère dépression, ce qui permet à l'utilisateur de saisir aisément un coin de l'opercule 5 pour procéder à son enlèvement.

L'opercule 5 est par exemple un film en matière plastique ou en métal ou un film composite comportant une ou plusieurs matières plastiques et/ou une ou plusieurs couches de métal.

Le réceptacle 2 se présente dans l'exemple considéré sous la forme d'une plaquette réalisée par thermoformage d'une feuille de matière plastique transparente, par exemple une polyoléfine.

Le volume du logement 3 est suffisant pour contenir, outre le produit 4, une quantité d'un liquide destiné à imprégner ce produit 4, suffisante pour permettre l'utilisation recherchée du produit 4.

Le volume du logement 3 est, par exemple, au moins le double du volume occupé par le produit 4 à l'intérieur de celui-ci. La profondeur du logement 3 est, dans l'exemple illustré, d'environ 2 ou 3 mm.

Pour utiliser le dispositif 1, l'utilisateur procède à l'enlèvement de l'organe de fermeture 5, puis verse le liquide dans le logement 3, le remplissage du logement 3 pouvant par exemple s'effectuer jusqu'à ce que le liquide recouvre entièrement le produit 4 ou soit sur le point de déborder du logement 3 ou encore, dans une variante non illustrée, jusqu'à une limite prédéfmie matérialisée par un trait sur le réceptacle, par exemple.

Lorsque le liquide est contenu dans un récipient, un doseur peut être prévu le cas échéant. Le liquide peut encore être contenu dans un récipient monodose et l'intégralité du contenu de ce récipient peut alors être versée dans le logement 3.

Le produit 4 comporte, conformément à l'invention, au moins un matériau superabsorbant se transformant en un gel, comme indiqué plus haut, et le cas échéant un ou plusieurs actifs, notamment choisis parmi ceux énumérés précédemment. Le produit 4 peut, notamment, se présenter sous forme d'un support fibreux.

Dans l'exemple des figures 1 et 2, le produit 4 étant, par exemple, destiné à exercer une action antiride sur le contour de l'oeil est, par exemple, un aiguilleté à base de fibres Hydrofiber de CONVATEC de 90 g/m$^2$, découpé sous la forme d'un patch réniforme de 6,5 cm dans sa plus grande longueur et de 2,3 cm dans sa plus grande largeur. Il peut être imprégné juste avant d'être appliqué au niveau du contour de l'oeil par environ 3 à 4 ml de la composition suivante : Eau qsp 100, extrait de feuilles de menthe en solution aqueuse 1 %, palmitate de rétinyle encapsulé 1 %, laponite 0,5 %, conservateurs 0,3 %, parfum 0,1 % (les proportions étant exprimées en masse).

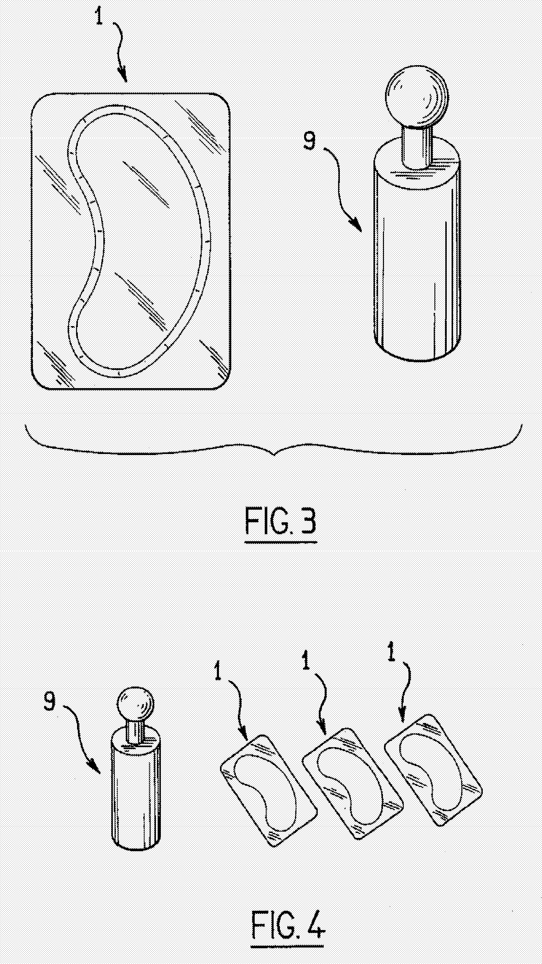

Sur la figure 3, on a représenté un kit comportant un dispositif 1 tel que décrit précédemment et un récipient 9 contenant le liquide destiné à imprégner le produit 4, le liquide étant par exemple l'un de ceux énumérés ci-dessus, pouvant comporter un ou plusieurs actifs cosmétiques et/ou dermatologiques.

La contenance du récipient 9 peut être adaptée à un usage unique ou, en variante, être adaptée à plusieurs utilisations.

Dans la variante illustrée à la figure 4, un récipient 9 est associé à une pluralité de dispositifs 1 afm, par exemple, de permettre des utilisations successives.

Les produits 4 contenus dans les dispositifs 1 peuvent être identiques ou, en variante, contenir des actifs différents de manière à permettre à l'utilisateur de choisir le dispositif 1 qu'il souhaite utiliser parmi plusieurs, en fonction du ou des actifs qu'il contient, selon le traitement à effectuer.

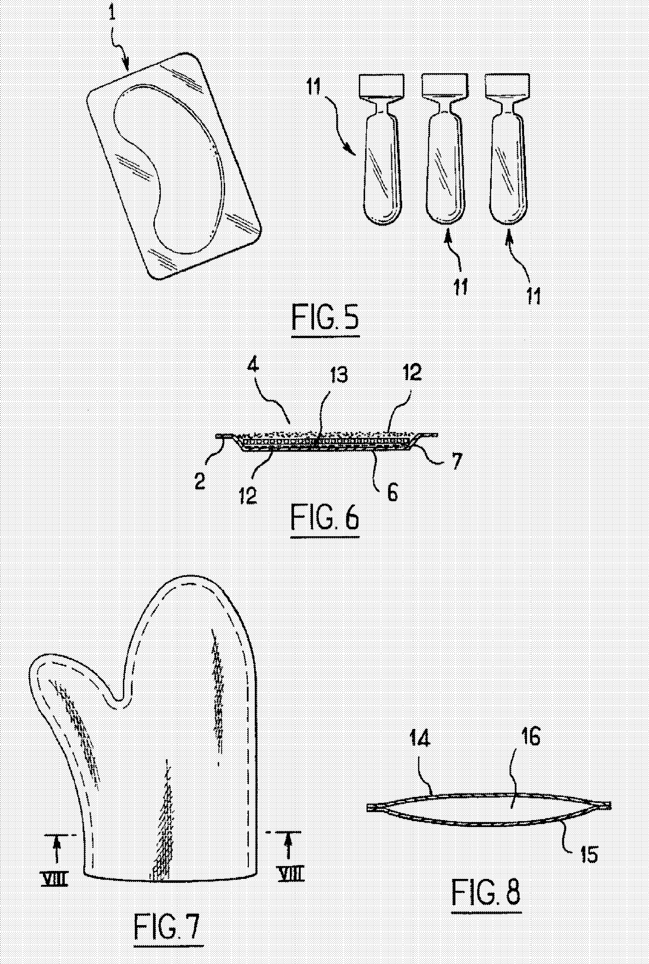

Dans la variante de réalisation de la figure 5 le kit comporte, outre le dispositif 1, plusieurs récipients 11 contenant des liquides respectifs différents, par exemple des liquides contenant des actifs spécifiques, ce qui permet à l'utilisateur de choisir par exemple le récipient 11 en fonction des actifs qu'il contient, selon le traitement à effectuer.

Les récipients 11 sont, par exemple, des flacons monodoses comportant un embout sécable, comme illustré. Bien entendu, les récipients 11 pourraient être autres, sans que l'on sorte du cadre de la présente invention, par exemple des ampoules.

La contenance du récipient 9 ou d'un récipient 11 est, par exemple, comprise entre 1 et 50 ml, notamment entre 3 et 30 ml, voire entre 4 et 20 ml.

Dans une variante non illustrée, le kit comporte plusieurs dispositifs 1 et plusieurs récipients 9 ou 11, les dispositifs étant identiques ou différents et les récipients identiques ou différents. Le kit peut, par exemple, comporter plusieurs dispositifs contenant différents actifs et plusieurs récipients contenant également différents actifs, l'utilisateur pouvant effectuer toute combinaison d'actifs de son choix.

Dans les exemples qui précèdent, le produit 4 peut se présenter sous la forme d'un patch comportant des fibres d'un matériau superabsorbant formant un gel, mais il pourrait en être autrement.

A titre d'illustration, on a représenté à la figure 6 une variante de réalisation dans laquelle le produit 4 comporte une poudre 12 du matériau superabsorbant formant un gel et un filet 13 disposé à l'intérieur de celle-ci.

Lors de l'imprégnation par le liquide, la poudre 12 se gélifie et la présence du filet permet d'assurer la cohésion du produit 4 et facilite également sa préhension.

Dans l'exemple de la figure 8, le produit présente une structure creuse, étant formée par l'assemblage de deux feuilles 14 et 15 définissant entre elles une cavité 16 dans laquelle peut être engagée une partie du corps, par exemple la main.

Le gant illustré figure 7 peut, par exemple, être destiné à traiter la main ou être destiné à permettre à l'utilisateur de traiter toute région du corps en utilisant le produit à la manière d'un gant de toilette.

Le gant, par exemple, destiné à l'application d'un auto-bronzant, est, par exemple, réalisé avec un non-tissé lié par jets d'eau à base de fibres Hydrofiber de CONVATEC de 50 g/m$^2$. Il est préalablement imprégné par du dihydroxyacétone en poudre à raison de 150 % en poids de dihydroxyacétone pour 100 g de support, par tout moyen adapté, notamment et de manière préférée par imprégnation par champ électrique alternatif.

Il peut être imprégné, juste avant de l'appliquer sur le corps, par environ 50 ml d'eau. Il peut aussi être utilisé directement sur la peau mouillée, sans le mouiller, en la massant de préférence de manière homogène pour révéler la coloration de manière uniforme.

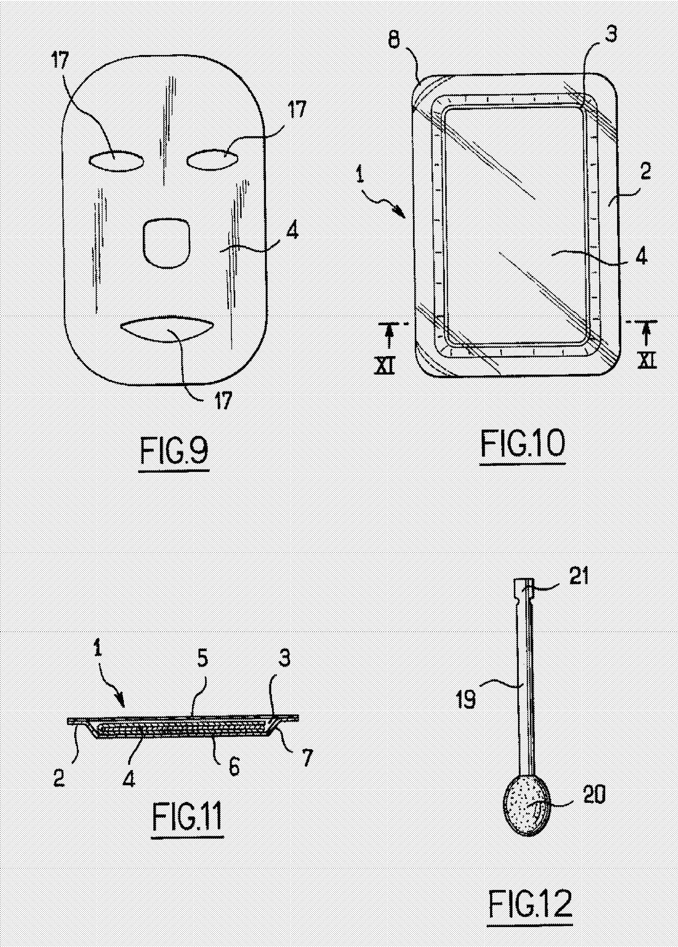

Le produit 4, lorsqu'il se présente sous la forme d'une structure creuse ou d'un masque ayant des ouvertures 17, tel qu'illustré figure 9, peut être conditionné dans une plaquette 2 à l'instar du mode de réalisation des figures 1 et 2, à l'état enroulé ou plié.

Le masque de la figure 9, par exemple, destiné à exercer une action anti-ride et favorisant l'éclat du teint, est, par exemple, un aiguilleté à base de fibres Hydrofiber de CONVATEC de 90 g/m$^2$ découpé en forme de visage de 20 cm dans sa plus grande hauteur et de 23 cm dans sa plus grande largeur. Il peut être préalablement imprégné par de l'acide ascorbique en poudre à raison de 150 % en poids de vitamine C pour 100 g de support, par tout moyen adapté, notamment et de manière préférée par imprégnation par champ électrique alternatif. Le masque peut être imprégné, juste avant de l'appliquer sur le visage, par environ 20 à 30 ml de la composition suivante : Eau qsp 100, allantoïne 1 %, glycérine 3 %, conservateurs 0,3 %, parfum 0,1 % (les proportions étant exprimées en masse).

L'utilisation de fibres d'un matériau superabsorbant capable de se gélifier en présence d'un liquide peut s'avérer avantageuse non seulement pour réaliser des produits 4 en feuille(s) destinés à être appliqués sur le corps humain, mais également pour réaliser des éléments d'application tels que celui illustré à la figure 12.

On a représenté sur cette figure, un applicateur comportant intérieurement au moins un liquide et, à une extrémité de ce tube, un élément d'application 20 pouvant être imprégné par le ou les liquides contenus dans le tube 19 lors de l'utilisation.

L'applicateur représenté à la figure 12 est par exemple identique, hormis la nature de l'élément d'application 20, à l'un de ceux décrits dans les demandes de brevet FR 2 845 005, FR 2 844 986, FR 2 844 973, FR 2 845 069 et FR 2 844 974 dont le contenu est incorporé à la présente demande par référence.

Le tube 19 comporte par exemple, à l'extrémité opposée à l'élément d'application 20, un embout sécable 21 qui lorsqu'il est rompu permet une entrée d'air dans le tube et l'écoulement du ou des liquides contenus à l'intérieur de celui-ci vers l'élément d'application 20.

Le cas échéant, le ou les liquide(s) contenus dans le tube 19 et l'élément d'application 20 peuvent être séparés par un bouchon d'un liquide, par exemple du silicone.

Lors de l'utilisation, l'utilisateur rompt la partie sécable 21 et le ou les liquide(s) contenus dans le tube 19 gagnent l'élément d'application 20.

De par la présence du matériau superabsorbant dans celui-ci, l'élément d'application 20 est capable d'absorber en totalité le ou les liquide(s) contenus dans le tube 19 et le risque d'égouttement du liquide imprégnant l'élément d'application 20 est réduit.

Le ou les liquide(s) contenus dans le tube 19 peuvent, par exemple, comporter au moins l'un des actifs cités précédemment.

La réalisation de l'élément d'application 20 avec des fibres d'un matériau superabsorbant formant un gel, tel que l'un de ceux énumérés plus haut, est particulièrement avantageuse lorsque le ou les produit(s) destinés à imprégner l'embout sont de faible viscosité.

L'élément d'application 20 peut être constitué essentiellement de fibres du matériau superabsorbant formant un gel, le cas échéant.

Dans une variante, l'élément d'application 20 ne comporte pas des fibres d'un matériau superabsorbant mais, par exemple, une poudre de ce matériau, laquelle est par exemple mélangée à des fibres de coton ou d'un autre matériau ou incorporée dans une structure alvéolaire ou un feutre, par exemple.

Le volume de liquide contenu dans le tube 19 est par exemple compris entre 0,01 et 5 ml, notamment entre 0,5 et 1 ml.

L'expression "comportant un" doit être comprise comme étant le synonyme de "comportant au moins un", sauf si le contraire est spécifié, et "compris entre" doit se comprendre bornes incluses.

**Revendications**

1. Dispositif (1) comportant :

 - un produit (4), notamment sous forme d'un support fibreux, destiné à être imprégné d'un liquide et à être appliqué sur le corps humain, comportant un matériau superabsorbant formant un gel après absorption du liquide,
 - un réceptacle (2) comportant un logement (3)

dans lequel est reçu le produit avant l'utilisation, le volume du logement (3) étant suffisant pour recevoir simultanément le produit et une quantité suffisante de liquide pour gélifier le produit (4).

2. Dispositif selon la revendication 1, **caractérisé en ce que** le matériau superabsorbant (4) comporte des fibres.

3. Dispositif selon la revendication 2, **caractérisé en ce que** les fibres sont liées entre elles autrement que par tissage.

4. Dispositif selon la revendication 2, **caractérisé en ce que** les fibres sont tissées.

5. Dispositif selon l'une quelconque des revendications 2 à 4, **caractérisé en ce que** les fibres en matériau superabsorbant constituent au moins 80 % en poids du produit (4) avant imprégnation par le liquide.

6. Dispositif selon l'une quelconque des revendications 2 à 4, **caractérisé en ce que** les fibres en matériau superabsorbant constituent au moins 90 % en poids du produit (4) avant imprégnation par le liquide.

7. Dispositif selon l'une quelconque des revendications 2 à 4, **caractérisé en ce que** les fibres en matériau superabsorbant constituent au moins 99 % en poids du produit (4) avant imprégnation par le liquide.

8. Dispositif selon l'une quelconque des revendications précédentes, **caractérisé en ce que** le matériau superabsorbant est choisi parmi les dérivés de cellulose, les alginates et leurs dérivés, les dérivés d'acide polyacrylique, ou polyméthacrylique, les dérivés de polyacrylamide, les dérivés de polyvinylpyrrolidonne, les dérivés d'éther polyvinylique, les gommes et leurs mélanges.

9. Dispositif selon la revendication précédente, **caractérisé en ce que** le produit (4) comporte un dérivé de cellulose en une teneur d'au moins 0,1 %, notamment au moins 10 %, 50 % ou 80 %, voire sensiblement égale à 100 %, en poids relativement au poids total du produit.

10. Dispositif selon la revendication 9, **caractérisé en ce que** le dérivé de cellulose comporte de la carboxyméthylcellulose.

11. Dispositif selon l'une quelconque des revendications précédentes, **caractérisé en ce que** le produit comporte au moins un actif cosmétique et/ou dermatologique.

12. Dispositif selon la revendication 11, **caractérisé en ce que** le produit comporte un auto-bronzant.

13. Dispositif selon la revendication 11, **caractérisé en ce que** le produit comporte un anti-ride.

14. Dispositif selon la revendication 11, **caractérisé en ce que** le produit comporte de la vitamine C.

15. Dispositif selon l'une quelconque des revendications 11 à 14, **caractérisé en ce que** le produit comporte un actif pulvérulent.

16. Dispositif selon la revendication 15, **caractérisé en ce que** l'actif pulvérulent a été incorporé au produit par un champ électrique alternatif

17. Dispositif selon l'une quelconque des revendications 11 à 16, **caractérisé en ce que** l'actif est présent dans une proportion en poids par rapport au poids total du produit sans l'actif supérieure ou égale à 100 %, notamment environ 150 %.

18. Dispositif selon l'une quelconque des revendications précédentes, **caractérisé en ce que** le produit (4) est agencé sous la forme d'au moins une feuille.

19. Dispositif selon la revendication 1, **caractérisé en ce que** le produit (4) comporte une poudre (12) et un filet (13) au contact de celle-ci.

20. Dispositif selon l'une quelconque des revendications précédentes, **caractérisé en ce que** le produit (4) présente une structure multicouche dont au moins une couche comporte le matériau superabsorbant.

21. Dispositif selon l'une quelconque des revendications précédentes, **caractérisé en ce que** le produit (4) comporte en outre un matériau sensiblement non absorbant vis-à-vis du liquide.

22. Dispositif selon l'une quelconque des revendications précédentes, **caractérisé en ce que**, après absorption du liquide, le produit (4) subit une diminution de sa surface extérieure d'au moins 10 %.

23. Dispositif selon l'une quelconque des revendications précédentes, **caractérisé en ce que**, après absorption du liquide, le produit présente une augmentation de son épaisseur d'au moins 50 %.

24. Dispositif selon l'une quelconque des revendications précédentes, **caractérisé en ce que** le produit présente une cohésion au moins après imprégnation par le liquide.

25. Dispositif selon l'une quelconque des revendications précédentes, **caractérisé en ce que** le produit (4) devient transparent ou translucide après imprégnation par le liquide.

26. Dispositif selon l'une quelconque des revendications précédentes, **caractérisé en ce que** le produit, après imprégnation par le liquide, est sensiblement non élastiquement déformable.

27. Dispositif selon l'une quelconque des revendications précédentes, **caractérisé en ce que** le produit (4) comporte une cavité (16) dans laquelle peut être engagée au moins une partie du corps humain, le produit se présentant notamment sous la forme d'un gant, d'un doigt de gant, d'une charlotte, d'une cagoule, d'un chausson, d'une poche à disposer autour d'une oreille.

28. Dispositif selon l'une quelconque des revendications 1 à 26, **caractérisé en ce que** le produit se présente sous la forme d'un patch ou d'un masque.

29. Dispositif selon l'une quelconque des revendications précédentes, **caractérisé en ce qu'**il comporte un organe de fermeture (5) du réceptacle (2).

30. Dispositif selon la revendication 29, **caractérisé en ce que** l'organe de fermeture (5) ferme de manière étanche le logement (3).

31. Dispositif selon l'une des revendications 29 et 30, **caractérisé en ce que** l'organe de fermeture (5) comporte un opercule.

32. Dispositif selon la revendication 31, **caractérisé en ce que** l'opercule (5) est fixé au réceptacle par collage ou thermosoudage.

33. Dispositif selon l'une quelconque des revendications précédentes, **caractérisé en ce que** le réceptacle (2) comporte une plaquette.

34. Dispositif selon l'une quelconque des revendications précédentes, **caractérisé en ce que** le logement (3) présente une paroi de fond (6) sensiblement plane.

35. Dispositif selon la revendication précédente, **caractérisé en ce que** le logement présente une paroi latérale (7) inclinée s'évasant en éloignement de la paroi de fond 6.

36. Dispositif selon la revendication précédente, **caractérisé en ce que** la paroi latérale 7 suit sensiblement le contour du produit (4) disposé dans le logement (3).

37. Dispositif selon l'une quelconque des revendications précédentes, **caractérisé en ce que** la profondeur du logement (3) est supérieure à l'épaisseur maximale du produit.

38. Dispositif selon l'une quelconque des revendications précédentes, **caractérisé en ce que** la profondeur du logement (3) est comprise entre 2 et 6 mm.

39. Dispositif selon l'une quelconque des revendications précédentes, **caractérisé en ce que** le logement (3) contenant le produit est l'unique logement du réceptacle (2).

40. Dispositif selon l'une quelconque des revendications précédentes, **caractérisé en ce que** le réceptacle (2) est au moins partiellement transparent.

41. Dispositif selon l'une quelconque des revendications précédentes, **caractérisé en ce que** le réceptacle (2) est obtenu par thermoformage.

42. Dispositif (1) comportant :

   - un produit (4) destiné, notamment sous forme d'un support fibreux, à être imprégné d'un liquide et à être appliqué sur le corps humain, comportant un matériau superabsorbant formant un gel après imprégnation avec le liquide, le produit (4) devenant alors translucide ou transparent,
   - un réceptacle (2) comportant un logement (3) dans lequel est reçu le produit.

43. Dispositif (1) comportant :

   - un produit (4) en feuille destiné à être gélifié au moyen d'un liquide et à être appliqué sur le corps humain,
   - un réceptacle (2) comportant un logement (3) dans lequel est reçu le produit (4), ce logement (3) présentant un volume suffisant pour contenir la quantité de liquide nécessaire pour la gélification du produit en feuille, le réceptacle (2) étant dépourvu de réservoir contenant ledit liquide avant l'étape de gélification.

44. Dispositif selon l'une quelconque des revendications précédentes, **caractérisé en ce qu'**il comporte plusieurs produits (4).

45. Dispositif selon la revendication précédente, **caractérisé en ce qu'**au moins deux produits contiennent des actifs différents.

46. Dispositif selon la revendication 44 ou 45, **caractérisé en ce que** les produits (4) sont contenus dans des logements (3) différents du réceptacle (2).

47. Dispositif selon la revendication 44 ou 45, **caractérisé en ce que** les produits (4) sont contenus dans des logements (3) différents appartenant respectivement à des réceptacles (2) différents.

**48.** Dispositif selon la revendication précédente, **caractérisé en ce que** les réceptacles (2) sont reliés deux à deux par une zone sécable.

**49.** Kit comportant :

- un dispositif (1) tel que défmi dans l'une quelconque des revendications précédentes, et
- un liquide destiné à imprégner le ou les produits (4).

**50.** Kit selon la revendication précédente, **caractérisé en ce qu'**il comprend une pluralité de liquides différents.

**51.** Kit selon l'une des revendications 49 ou 50, **caractérisé en ce que** le ou chaque liquide est conditionné dans un récipient monodose (12).

**52.** Kit comportant :

- un produit (4) destiné à être imprégné d'un liquide et à être appliqué sur le corps humain, comportant au moins 80 % en poids d'un matériau superabsorbant formant un gel après absorption du liquide, et
- le liquide destiné à gélifier ledit produit.

**53.** Produit en feuille (4) destiné à être imprégné d'un liquide et à être appliqué sur le corps humain, comportant au moins 80 % en poids d'un matériau superabsorbant autre que le polyvinylalcool et formant un gel après absorption du liquide.

**54.** Applicateur comportant :

- un tube (19) rempli d'au moins un liquide,
- un élément d'application (20) destiné à être imprégné par le ou les liquide(s) contenu(s) dans le tube et à être appliqué sur le corps humain, comportant un matériau superabsorbant.

**55.** Procédé de traitement cosmétique, comportant les étapes suivantes :

- imprégner d'un liquide un produit (4) comportant au moins 80 % en masse d'un matériau superabsorbant, autre que le polyvinyl alcool, formant un gel après absorption du liquide, et
- mettre en contact une surface à traiter avec le produit (4) gélifié.

**56.** Procédé de traitement cosmétique comportant les étapes suivantes :

- verser dans le logement (3) d'un dispositif (1) tel que défini dans l'une quelconque des revendications 1 à 43 un liquide pour imprégner le produit (4), et
- mettre en contact une surface à traiter avec le produit (4) ainsi imprégné.

FIG.1

FIG.2

FIG. 3

FIG.4

FIG.5

FIG. 6

FIG.7

FIG. 8

FIG.9

FIG.10

FIG.11

FIG.12

**Office européen
des brevets**

**RAPPORT DE RECHERCHE EUROPEENNE**

Numéro de la demande

EP 05 30 0815

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | FR 2 781 667 A (L'OREAL) 4 février 2000 (2000-02-04)<br><br>* page 10, alinéa 2 - alinéa 3; revendications 21-29; figures 1,2; exemple 3 *<br>* page 2, ligne 19 - ligne 20 *<br>----- | 1,8,10, 11,16, 21-24, 26, 28-42, 49-51 | A61L15/28 A61K8/73 A61K8/02 A45D44/00 A61J1/00 A61M35/00 B65D81/32 |
| X | DE 44 46 380 A1 (LTS LOHMANN THERAPIE-SYSTEME GMBH & CO KG, 56567 NEUWIED, DE) 11 janvier 1996 (1996-01-11)<br><br>* colonne 2, ligne 25 - ligne 34; revendications 1,7; figures 1,2; exemple 1 *<br>* colonne 2, ligne 48 - ligne 43 *<br>----- | 1,8,11, 13,16, 21-26, 28,42, 49,55 | |
| D,X<br><br>Y | WO 95/19795 A (BRISTOL-MYERS SQUIBB COMPANY; LYDON, MICHAEL, JAMES; QUEEN, DOUGLAS) 27 juillet 1995 (1995-07-27)<br>* revendications 1,5,6,8; exemples 1-5 *<br><br>----- | 52,53<br><br>1-51,54, 55 | **DOMAINES TECHNIQUES RECHERCHES (IPC)**<br>A61L A61K A45D A61M A61J B65D |
| D,X<br><br>Y | WO 02/03898 A (BRISTOL-MYERS SQUIBB COMPANY; GRIFFITHS, BRYAN; PRITCHARD, DAVID, COLI) 17 janvier 2002 (2002-01-17)<br>* revendications 1-9; exemples 1-3 *<br><br>----- | 52,53<br><br>1-51,54, 55 | |
| D,Y | EP 0 737 463 A (GARCONNET, MICHEL; GARCONNET, CLAUDINE MARIE-CHRISTINE; GARCONNET, ARM) 16 octobre 1996 (1996-10-16)<br>* abrégé; figures 1-4 *<br>----- | 1-51,54, 55 | |

-/--

Le présent rapport a été établi pour toutes les revendications

| Lieu de la recherche | Date d'achèvement de la recherche | Examinateur |
|---|---|---|
| Munich | 3 mars 2006 | Laffargue-Haak, T |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P04C02)

**Office européen des brevets**

**RAPPORT DE RECHERCHE EUROPEENNE**

Numéro de la demande

EP 05 30 0815

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | DE 102 58 992 A1 (BEISEL, GUENTHER) 8 juillet 2004 (2004-07-08) * alinéas [0011], [0064], [0066], [0099] - [0106]; figures 1,2 * ----- | 1-51,54, 55 | |
| | | | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 3 mars 2006 | Laffargue-Haak, T |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 05 30 0815

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-03-2006

| Document brevet cité au rapport de recherche | | | Date de publication | Membre(s) de la famille de brevet(s) | | | Date de publication |
|---|---|---|---|---|---|---|---|
| FR 2781667 | A | | 04-02-2000 | BR | 9903345 | A | 06-06-2000 |
| | | | | CA | 2279121 | A1 | 30-01-2000 |
| | | | | CN | 1252272 | A | 10-05-2000 |
| | | | | DE | 69909029 | D1 | 31-07-2003 |
| | | | | DE | 69909029 | T2 | 06-05-2004 |
| | | | | EP | 0976382 | A1 | 02-02-2000 |
| | | | | ES | 2201602 | T3 | 16-03-2004 |
| | | | | JP | 3462804 | B2 | 05-11-2003 |
| | | | | JP | 2000086496 | A | 28-03-2000 |
| DE 4446380 | A1 | | 11-01-1996 | AUCUN | | | |
| WO 9519795 | A | | 27-07-1995 | AT | 235925 | T | 15-04-2003 |
| | | | | AU | 691245 | B2 | 14-05-1998 |
| | | | | AU | 1667395 | A | 08-08-1995 |
| | | | | CA | 2179415 | A1 | 27-07-1995 |
| | | | | DE | 69530180 | D1 | 08-05-2003 |
| | | | | DE | 69530180 | T2 | 08-01-2004 |
| | | | | DK | 740554 | T3 | 07-07-2003 |
| | | | | EP | 0740554 | A1 | 06-11-1996 |
| | | | | ES | 2193190 | T3 | 01-11-2003 |
| | | | | FI | 962916 | A | 19-07-1996 |
| | | | | JP | 9509590 | T | 30-09-1997 |
| | | | | NO | 962316 | A | 03-07-1996 |
| | | | | NZ | 279516 | A | 28-07-1998 |
| | | | | PT | 740554 | T | 30-06-2003 |
| | | | | US | 6471982 | B1 | 29-10-2002 |
| WO 0203898 | A | | 17-01-2002 | AU | 782292 | B2 | 14-07-2005 |
| | | | | AU | 7080401 | A | 21-01-2002 |
| | | | | CA | 2383915 | A1 | 17-01-2002 |
| | | | | EP | 1299058 | A1 | 09-04-2003 |
| | | | | GB | 2370781 | A | 10-07-2002 |
| | | | | JP | 2004502496 | T | 29-01-2004 |
| | | | | NZ | 517608 | A | 29-08-2003 |
| | | | | US | 2002038099 | A1 | 28-03-2002 |
| EP 0737463 | A | | 16-10-1996 | AT | 186459 | T | 15-11-1999 |
| | | | | AU | 702273 | B2 | 18-02-1999 |
| | | | | AU | 5053696 | A | 24-10-1996 |
| | | | | BR | 9601306 | A | 13-01-1998 |
| | | | | CA | 2173801 | A1 | 11-10-1996 |
| | | | | CN | 1143038 | A | 19-02-1997 |
| | | | | DE | 69605074 | D1 | 16-12-1999 |
| | | | | EG | 20817 | A | 29-03-2000 |
| | | | | FR | 2732585 | A1 | 11-10-1996 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE
RELATIF A LA DEMANDE DE BREVET EUROPEEN NO.**

EP 05 30 0815

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-03-2006

| Document brevet cité au rapport de recherche | | Date de publication | Membre(s) de la famille de brevet(s) | | Date de publication |
|---|---|---|---|---|---|
| EP 0737463 | A | | IL 117772 A | | 22-09-1999 |
| | | | JP 9020343 A | | 21-01-1997 |
| | | | OA 10281 A | | 07-10-1997 |
| | | | US 5766715 A | | 16-06-1998 |
| | | | ZA 9602704 A | | 09-10-1996 |
| DE 10258992 | A1 | 08-07-2004 | AUCUN | | |

EPO FORM P0460

Pour tout renseignement concernant cette annexe : voir Journal Officiel de l'Office européen des brevets, No.12/82